# EUROPEAN PATENT SPECIFICATION

(11) **EP 3 359 198 B1**
(45) Date of publication and mention of the grant of the patent: **13.05.2020**
(21) Application number: 16791471.2
(22) Date of filing: 05.10.2016
(51) Int. Cl.: A61K 47/10

(54) **TOPICAL MINOXIDIL COMPOSITION**
TOPISCHE MINOXIDIL-ZUSAMMENSETZUNG
COMPOSITION DE MINOXYDILE TOPIQUE

(30) Priority: 06.10.2015 TR 201512369; 18.10.2015 TR 201512927
(43) Date of publication of application: 15.08.2018
(73) Proprietor: Assos Ilaç Kimya Gida Ürünleri Üretim Ve Tic. A.S., 34773 Ümraniye/Istanbul (TR)
(72) Inventor: NURIOGULLARI, Eymen, Maltepe/Istanbul (TR)
(74) Representative: Bulut, Pinar
(86) International application number: PCT/TR2016/050369
(87) International publication number: WO 2017/061971

(56) References cited:
- EP-A2- 0 249 397
- WO-A1-2015/095181
- JP-A- 2007 191 473
- US-A1- 2011 189 115

## Description

### Technical Field

The present invention is related to a topical Minoxidil foam composition, wherein the excipient 1.3- propanediol used for the first time as a solvent can be substituted with polyethylene glycol and an exemplary production method of Minoxidil foam or spray composition using 1.3-propanediol.

### Prior Art

The molecule name of Minoxidil is 3-hidroxy-2-imino-6-piperidine-1-pyrimidine-4-amine and the molecule formula has been shown below.

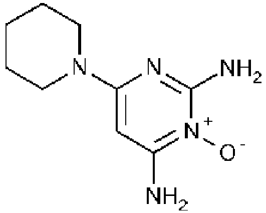

Minoxidil has been mentioned to be used in the treatment of alopecia as a spray or aerosol having different concentration topically in the patent numbered US6946120. In the WO/2009/101497A2 and WO/2009/101497A3 however, it is mentioned that Minoxidil is used in combination with Aminexil. However there is no other data regarding the usage of Minoxidil as a "spray" topically being used in alopecia treatment in these or other patents.

In WO9953923 the usage of Minoxidil in the form of foam is mentioned. In the patent numbered 6946120 which is a continuation of the afore mentioned patent, the usage of Minoxidil in 5% concentrations in foam form for the treatment of alopecia in men and women and the formulation used for this foam is described.

EP0249397 A2 relates to the penetration-enhancing composition of minoxidil. Penetration-enhancing carrier consist of a small polar solvent selected from 1,2-propanediol, 1,2-butanediol, 1,3-butanediol, 2,3-butanediol, C₃-C₆ triols, or mixtures thereof, and a polar lipid compound selected from oleyl alcohol, isocetyl alcohol, and mixtures thereof.

WO2015/095181 A1 related to the a topical composition of minoxidil, at least one polymer including a poly(monostearoyl glycerol-co- succinate) polymer; at least one lower alcohol; and at least one co-solvent. The glycols as co-solvent are selected from the group consisting of propylene glycol, ethylene glycol, butylene glycol, 1,2-butanediol, 1,3-butanediol, 1,4-butanediol, 2,3-butanediol, pentylene glycol, hexylene glycol, propanediol, propanediol, dipropylene glycol, ethoxydiglycol, methylpropanediol, isopentyldiol, and mixtures thereof.

Minoxidil is a peripheric vasodilator agent. It causes reduction in blood pressure by reducing peripheric resistance. At the same time it is also used as an adrogenetic alopecia topical product in women and men (to revitalize areas without hair). It is used topically between 1-5% (wt/wt). Products having pharmaceutical forms such as tablet, solution, spray, and foam are being sold in America and Europe, comprising this active ingredient.

### Detailed Description of the Invention

In the known state of the art a topical spray product comprising Minoxidil and a production method belonging to said spray product to be used in the treatment of alopecia is described in the patent numbered US 4,828,837. In this formula the form of Minoxidil comprising an amphipatic compound lower than pK 5 and the content of sulphate, phosphonate, sulphonate inside a single half ring lipophylic compound which is not crystalline in aqueous form has been defined. However in none of the patent data (for all Minoxidil forms) 1,3- propanediol has not been mentioned as a solvent.

In the known state of the art a topical foam product comprising Minoxidil and a production method belonging to said foam product to be used in the treatment of alopecia is described in the patent numbered US 6946120 and WO9953923. The use of alcohol, water, propylene glycol and polyethylene glycol as solvent is specified in these patents. Particularly, it is stated that the ratio of alcohol:water is 1:1 to 1:3. However in none of the patent data (for all Minoxidil forms) 1,3- propanediol has not been mentioned as a solvent.

The scope of the invention is defined by the claims. As a result of the studies that have been carried out it has been found out that Minoxidil in suitable solvent/solvents could be used at a concentration between 1%-5% (weight/weight) and that the topical foam solution did not convey any stability or incompatibility problem.
Minoxidil is a molecule that is poorly dissolved inside water. It is dissolved at a rate of 2200mg/L in water (Merck Index). For this reason, in the case that only water is used as a solvent, a sufficient amount of solubility has not been able to be obtained for even the lowest Minoxidil concentration. If a Minoxidil solution at a concentration higher than 1% is desired to be prepared, solubility problem is faced.

Sufficient amount of solubility can be obtained even at the highest concentration (5%) that can be used in propylene glycol, methanol, ethanol and 2-propanol. However the best absorption for Minoxidil is for it to be used in ethanol or a mixture of ethanol/propylene glycol. Moreover it has been noted that it can be dissolved in a suitable dissolving agent such as alcohol (propylene glycol, methanol, ethanol, 2-propanol) and other co-solvent such as some aromatic and polyhydric alcohols (cetyl alcohol, stearyl alcohol, benzyl alcohol, polyoxyethylene lauryl alcohol, 1-3butylene glycol, glycerol). The usage of 1,3-propanediol has not been mentioned in any topical Minoxidil study. However it has been found that Minoxidil is soluble in 1,3-propanediol as much as it is soluble in propylene glycol.

The topical Minoxidil foam composition can comprise one or more excipients as co-solvent selected from ethanol, methanol, propylene glycol, polyethylene glycol, 2-propanol, cetyl alcohol, stearyl alcohol, dimethylsulphoxide, benzyl alcohol, glycerol, 1-3butylene glycol, polyoxy ethylene lauryl alcohol together with 1-3 propanediol. Preferably it comprises ethanol as co-solvent.

The general formulation not according to the invention of a spray composition comprising Minoxidil has been given below; wherein Minoxidil is used at a rate of 1-5%, surface active agent(s) at a rate of 10-20%, solubility agent(s) at a rate of 3-97% and an acidifying agent at a rate of 1-5%.

| **CONTENT** | **CONCENTRATION, %** |
|---|---|
| Minoxidil | 1 - 5 (wt/wt) |
| One or more solvents (including 1,3-propanediol) | 1 - 97 (wt/wt) |
| Acidifying agent | 1 - 5 (wt/wt) |
| Demineralised water | k.m. |
| Total | 100 |

| | |
|---|---|
| *wt/wt (weight/weight) | |

Said method suitable to the invention basically comprises the below mentioned steps:
a) Minoxidil is dissolved in a suitable solvent,
b) An acidifying agent is added into or dissolved in the final mixture
c) The solution is completed to its final volume with demineralised water or with the remaining amount of the solvent
d) The product is filtered
e) The end products are packaged.

The general formulation of a foam composition comprising Minoxidil has been given below; wherein Minoxidil is used at a rate of 1-5%, surface active agent(s) at a rate of 10-20%, solubility agent(s) at a rate of 3-97% and an acidifying agent at a rate of 1-5%.

| **CONTENT** | **CONCENTRATION, %** |
|---|---|
| Minoxidil | 1 - 5 (wt/wt) |
| Surface active agent(s) | 10 - 20 (wt/wt) |
| One or more solvents (including 1,3-propanediol) | 3 - 97 (wt/wt) |
| Acidifying agent | 1 - 5 (wt/wt) |
| Demineralised water | k.m. |
| Total | 100 |

| | |
|---|---|
| *wt/wt (weight/weight) | |

Said method suitable to the invention basically comprises the below mentioned steps:
a) Minoxidil is dissolved in a suitable solvent,
b) Surface active agent(s) is added into the solution,
c) An acidifying agent is added into or dissolved in the final mixture,
d) The solution is completed to its final volume with demineralised water or with the remaining amount of the solvent
e) The product is filtered,
f) The end products are packaged.

Topical foam compositions obtained according to a methods suitable to the invention show high solubility and did not pose any incompatibility problem. A stability problem was not encountered regarding the topical compositions obtained with a method suitable to the invention following the 3 and 6 month long term stability studies and 3 and 6 month accelerated stability studies. The topical Minoxidil composition can be sprayed with a suitable pump. The topical compositions according to the invention are efficient in alopecia treatment.

### Example 1: Minoxidil 5% Spray not according to the invention

| **Active agent** | **Amount (gr/100gr)** | |
|---|---|---|
| Minoxidil | 5 | Active Agent |

| **Excipients** | | |
|---|---|---|
| 1,3-Propanediol | 60,529 | Solvent/excipient (USP) |
| Ethyl alcohol | 10,166 | Solvent (EP) |
| Lactic Acid | 2,466 | Acidifying agent (EP) |
| Deionised water | 21,779 | Solvent (EP) |
| Mint Essence | 0,06 | Aroma/Essence (EP) |

The production flow scheme of the formulation is as follows:
1- Minoxidil and other excipients are separately weighed and prepared,
2- 1,3-Propanediol, Lactic Acid, Ethyl Alcohol and Deionised Water which have been respectively weighed are placed in a production vessel and are mixed for at least 5 minutes,
3- The previously weighed Minoxidil is placed in a stainless steel production vessel and is mixed for at least 15 minutes in order to be dissolved,
4- Mint essence is added and mixed,
5- Filling is carried out at target volume and the end product is packaged and boxed.

### Example 2: Minoxidil 2% Spray not according to the invention

| **Active Agent** | **Amount (gr/100gr**) | |
|---|---|---|
| Minoxidil | 2 | Active Agent |

| **Excipients** | | |
|---|---|---|
| 1,3-Propanediol | 65,868 | Solvent/excipient (USP) |
| Ethyl Alcohol | 5,166 | Solvent (EP) |
| Lactic Acid | 2,466 | Acidifying agent (EP) |
| Deionised water | 24,44 | Solvent (EP) |
| Mint aroma | 0,06 | Aroma/Essence (EP) |

The production flow scheme of the formulation is the same as Example 1.

### Example 3: Minoxidil 5% Foam

| **Active agent** | **Amount (gr/100gr)** | |
|---|---|---|
| Minoxidil | 5 | Active Agent |

| **Excipients** | | |
|---|---|---|
| 1,3-Propanediol | 51,50 | Solvent/excipient (USP) |
| PEG 20 Oleyl Ether | 0,53 | Surface active agent |
| Plantacare 1200 * | 10,505 | Surface active agent |
| Komperlan KD ** | 3 | Surface active agent |
| Ethyl alcohol | 5,166 | Solvent (EP) |
| Lactic Acid | 2,466 | Acidifying agent (EP) |
| Deionised water | 21,779 | Solvent (EP) |
| Mint Essence | 0,06 | Aroma/Essence (EP) |

| | | |
|---|---|---|
| (*) Lauryl glycoside (*) Cocamide DEA = Coconut fatty Acid Diethanolamine | | |

The production flow scheme of the formulation is as follows:
1- Minoxidil and other excipients are separately weighed and prepared,
2- 1,3-Propanediol, Lactic Acid, Ethyl Alcohol and Deionised Water which have been respectively weighed are placed in a production vessel and are mixed for at least 5 minutes,
3- The previously weighed Minoxidil is placed in a stainless steel production vessel and is mixed for at least 15 minutes in order to be dissolved,
4- The previously weighed PEG 20 Oleyl Ether , Plantacare 1200 and Komperlan KD is added in a stainless steel production vessel and dissolved,
5- Mint essence is added and mixed,
6- Filling is carried out at target volume and the end product is packaged and boxed.

### Example 4: Minoxidil 2% Foam

| **Active Agent** | **Amount (gr/100gr)** | |
|---|---|---|
| Minoxidil | 2 | Active Agent |

| **Excipients** | | |
|---|---|---|
| 1,3-Propanediol | 51,833 | Solvent/excipient (USP) |
| PEG 20 Oleyl Ether | 0,53 | Surface active agent |
| Plantacare 1200 | 10,505 | Surface active agent |
| Komperlan KD | 3 | Surface active agent |
| Ethyl Alcohol | 5,166 | Solvent (EP) |
| Lactic Acid | 2,466 | Acidifying agent (EP) |
| Deionised water | 24,44 | Solvent (EP) |
| Mint aroma | 0,06 | Aroma/Essence (EP) |

The production flow scheme of the formulation is the same as Example 3.

### Results:

Alcohol (such as methanol, ethanol, propylene glycol, 2-propanol etc) or some aromatic and polyhydric alcohols as co-solvent together with alcohol (cetyl alcohol, stearyl alcohol, benzyl alcohol, polioxyethylene lauryl alcohol, 1,3-butylene glycol, glycerol) are used all around the world. However none of these worldwide examples comprise the usage of 1,3- propanediol as mentioned above.

As the product is used topically, it may lead to irritation at the application area if the product is used for a long period of time. The alcohol amount in the composition needed to be kept at a minimum. For this reason carrying out the dissolving process using minimum alcohol amounts such that stability is not disrupted and completing the final volume with water is more suitable in terms of usage. Therefore the alcohol/water or alcohol/water mixture within the composition needs to be combined at a suitable ratio. Particularly as the best Minoxidil absorption is observed in ethanol+propylene or ethanol+propylene glycol, and as 1,3-propanediol and propylene glycol shows similarities in terms of Minoxidil solubility, the best solvent composition for a combined product is ethanol+1,3-propanediol glycol mixture and water for completion to final volume. The alcohol/water or alcohols/water mixture can be at a ratio between 1:9 to 9:1 per volume. The best result has been obtained with a mixture of ethyl alcohol:water:1,3-propanediol at the ratio of 1:5,5:15, (wt/wt) for spray and the best result has been obtained with a mixture of ethyl alcohol:water:1,3-propanediol at the ratio of 1:4,5:10 (wt/wt) for foam composition.

If it is taken into consideration that the product obtained is used topically it needs to have a pH value below 7. For this reason, acidifying agents such as lactic acid, palmitic acid or a mixture thereof at any ratio is used. In all of the our studies the pH value was not lower than 4 and was not higher than 7. These values are suitable pH ranges for topical use.

Moreover if it is kept in mind that the composition obtained shall be applied to the skin up to 1% of aroma can be added to the product in order to avoid a bad scent that may be encountered.

The concentration of surface active agent may be from 10 to 20% for the formation of the foam of resulting solution. As the desired properties of the composition, the foam may be dissolve to the surface of the skin, but it should not be a very loose structure. According to this aim, the best results were obtained as 14% (w / w) of the amount of surface active agents in the experiments. It has also been observed in studies on; when the amount of minoxidil in the solution increased, the amount of the surface active agents required for the formation of the foam increases.

## Claims

1. A topical Minoxidil foam composition, **characterized in that** it comprises 1,3-propanediol as a solubilising agent, lactic acid as an acidifying agent at the ratio of 1 to 5% (wt/wt) by weight, and Lauryl Glucoside as a surface active agent at the ratio of 10 to 20% (wt/wt) by weight, ethanol and water; wherein ethyl alcohol: water: 1,3-propanediol is at a ratio of 1:4.5:10 by weight.

2. A topical Minoxidil foam composition according to claim 1, **characterized in that** the composition has a pH value between 4-7.

3. A topical Minoxidil foam composition according to claim 1, **characterized in that** it comprises 1 to 5% (wt/wt) Minoxidil.

4. A topical Minoxidil foam composition according to claim 3, **characterized in that** it comprises 2% (wt/wt) Minoxidil.

5. A topical Minoxidil foam composition according to claim 3, **characterized in that** it comprises 5% (wt/wt) Minoxidil.

6. A production method of a topical Minoxidil foam composition according to claim 1 **characterized in that** said method comprises the following steps:
a) Minoxidil and other excipients are separately weighed and prepared,
b) 1,3-Propanediol, Lactic Acid, Ethyl Alcohol and Deionised Water which have been respectively weighed are placed inside a production vessel and are mixed for at least 5 minutes,
c) The previously weighed Minoxidil is placed inside a stainless steel production vessel and is mixed for at least 15 minutes in order to be dissolved,
d) The previously weighed PEG 20 Oleyl Ether Lauryl glycoside and Coconut fatty Acid Diethanolamine is added in a stainless steel production vessel and dissolved,
e) Mint essence is added and mixed,
f) Filling is carried out at target volume and the end product is packaged and boxed.

7. A topical Minoxidil foam composition according to claim 1, **characterized in that** the composition can be used by means of a suitable pump.

8. A topical Minoxidil foam composition according to claims 1-5 or 7 for use in the treatment of alopecia.

## Patentansprüche

1. Topische Minoxidil-Schaumzusammensetzung, **dadurch gekennzeichnet, dass** sie 1,3-Propandiol als Lösungsvermittler, Milchsäure als Säuerungsmittel im Verhältnis von 1 bis 5% (Gew./Gew.) und Laurylglucosid als Tensid im Verhältnis von 10 bis 20% (Gew./Gew.), Ethylalkohol und Wasser umfasst, wobei Ethylalkohol: Wasser: 1,3-Propandiol im Verhältnis von 1:4.5:10, bezogen auf das Gewicht, vorliegt.

2. Topische Minoxidil-Schaumzusammensetzung nach anspruch 1, **dadurch gekennzeichnet, dass** die Zusammensetzung einen pH-Wert zwischen 4-7.

3. Topische Minoxidil-Schaumzusammensetzung nach anspruch 1, **dadurch gekennzeichnet, dass** sie 1 bis 5 % (Gew./Gew.) Minoxidil umfasst.

4. Topische Minoxidil-Schaumzusammensetzung nach anspruch 3, **dadurch gekennzeichnet, dass** sie 2% (Gew./Gew.) Minoxidil umfasst.

5. Topische Minoxidil-Schaumzusammensetzung nach anspruch 3, **dadurch gekennzeichnet, dass** sie 5% (Gew./Gew.) Minoxidil umfasst.

6. Verfahren zur Herstellung einer topischen Minoxidil-Schaumzusammensetzung nach anspruch 1, **dadurch gekennzeichnet, dass** das Verfahren die folgenden Schritte umfasst:
a) Minoxidil und andere Hilfsstoffe werden getrennt gewogen und vorbereitet,
b) 1,3-Propandiol, Milchsäure, Ethylalkohol und deionisiertes Wasser, die jeweils gewogen wurden, werden in ein Produktionsbehälter gegeben und mindestens 5 Minuten lang gemischt,
c) Das zuvor gewogene Minoxidil wird in ein Edelstahl-Produktionsbehälter gegeben und mindestens 15 Minuten lang gemischt, um sich aufzulösen,
d) Der zuvor gewogene PEG 20 Oleyl Ether, Lauryl Glucosid und Kokosnussfettsäure Diethanolamin wird in einem Edelstahl-Produktionsbehälter hinzugefügt und aufgelöst,
e) Minzessenz wird hinzugefügt und gemischt,
f) Die Abfüllung erfolgt mit dem Zielvolumen und das Endprodukt wird verpackt und geboxt.

7. Topische Minoxidil-Schaumzusammensetzung nach anspruch 1, **dadurch gekennzeichnet, dass** die Zusammensetzung mit Hilfe einer geeigneten Pumpe verwendet werden kann.

8. Topische Minoxidil-Schaumzusammensetzung nach den ansprüchen 1-5 oder 7 zur Verwendung bei der Behandlung von Alopezie.

## Revendications

1. Composition topique de mousse de Minoxidil, **caractérisée en ce qu'**il comprend du 1,3-propanediol comme un agent solubilisant, de l'acide lactique comme un agent acidifiant dans un rapport de 1 à 5% (poids/poids) en poids, et de Lauryl Glucoside comme un agent tensioactif dans un rapport de 10 à 20% (poids/poids) en poids, d'alcool éthylique et de l'eau ; où alcool éthylique : eau : 1,3-propanediol est dans un rapport de 1:4.5:10 en poids.

2. Composition topique de mousse de Minoxidil selon la revendication 1, **caractérisée en ce que** la composition a une valeur de pH comprise entre 4-7.

3. Composition topique de mousse de Minoxidil selon la revendication 1, **caractérisée en ce qu'**il contient de 1 à 5% (poids/poids) de Minoxidil.

4. Composition topique de mousse de Minoxidil selon la revendication 3, **caractérisée en ce qu'**il contient de 2% (poids/poids) de Minoxidil.

5. Composition topique de mousse de Minoxidil selon la revendication 3, **caractérisée en ce qu'**il contient de 5% (poids/poids) de Minoxidil.

6. Méthode de production d'une composition topique de mousse de Minoxidil selon la revendication 1, **caractérisée en ce que** ladite méthode comprend les étapes suivantes :
a) Le minoxidil et les autres excipients sont pesés et préparés séparément,
b) 1,3-propanediol, l'acide lactique, l'alcool éthylique et l'eau déionisée qui ont été respectivement pesés sont placés dans un récipient de production et sont mélangés pendant au moins 5 minutes,
c) Le minoxidil préalablement pesé est placé dans un récipient de production en acier inoxydable et est mélangé pendant au moins 15 minutes afin d'être dissous,
d) L'éther d'oléyle PEG 20, le lauryl glucoside et la diéthanolamine d'acide gras de noix de coco, préalablement pesés, sont ajoutés dans un récipient de production en acier inoxydable et dissous,
e) L'essence de menthe est ajoutée et mélangée,
f) Le remplissage est effectué au volume cible et le produit final est emballé et mis en boîte.

7. Composition topique de mousse de Minoxidil selon la revendication 1, **caractérisée en ce que** la composition peut être utilisée au moyen d'une pompe appropriée.

8. Composition de mousse de Minoxidil selon les revendications 1-5 ou 7 pour une utilisation dans le traitement de l'alopécie.
